# EUROPEAN PATENT APPLICATION

(11) **EP 3 838 292 A1**
(43) Date of publication of application: **23.06.2021**
(21) Application number: 20216082.6
(22) Date of filing: 28.01.2015
(51) Int. Cl.: A61K 47/50, A61K 9/00, A61K 9/14, A61K 9/51, A61K 38/17, A61P 17/00, A61P 27/02, A61P 43/00, A61K 47/10, A61K 47/12, A61K 47/14, A61K 47/32, A61K 47/64

(54) **TRANSMUCOSAL AND TRANSEPITHELIAL DRUG DELIVERY SYSTEM**

(30) Priority: 28.01.2014 GB 201401453
(62) Divisional of application: 15702553.7
(71) Applicant: MacRegen, Inc., Birmingham, Alabama 35233 (US)
(72) Inventor: Stamboulis, Artemis, Edgbaston, Birmingham, West Midlands B15 2TT (GB); De Cogan, Felicity Jane, Edgbaston, Birmingham, West Midlands B15 2TT (GB); Scott, Robert, Edgbaston, Birmingham, West Midlands B15 2TT (GB); Peacock, Anna Frances Acushla, Edgbaston, Birmingham, West Midlands B15 2TT (GB); Hill, Lisa Jayne, Edgbaston, Birmingham, West Midlands B15 2TT (GB)
(74) Representative: Hollah, Dorothee

(57) **Abstract**

The invention provides a transmembrane delivery system comprising:
A pharmaceutically active moiety; and
A polypeptide of up to 20 amino acids in length comprising a continuous region of at least 2, more typically at least 4 basic amino acids.

Typically, the system comprises a polypeptide, which has the formula:

(B)n (A)m

Where B is a basic amino acid
A is an acidic amino acid, and
m and n and integers and
n is at least 4
m is less than n.

SEQUENCE LISTING

<110> MacRegen, Inc.

<120> Transmucosal and Transepithelial Drug Delivery System

<130> M71915PCEPT1

<140> GB1401453.4
<141> 2014-01-28

<160> 7

<170> BiSSAP 1.3

<210> 1
<211> 6
<212> PRT
<213> Artificial Sequence

<220>
<223> peptide synthesis product

<400> 1
Arg Lys Leu Pro Asp Ala
1               5

<210> 2
<211> 10
<212> PRT
<213> Artificial Sequence

<220>
<223> peptide synthesis product

<400> 2
Arg Arg Arg Arg Arg Arg Arg Arg Arg Arg
1               5                   10

<210> 3
<211> 6
<212> PRT
<213> Artificial Sequence

<220>
<223> peptide synthesis product

<400> 3
Arg Arg Arg Arg Arg Arg
1               5

<210> 4
<211> 6
<212> PRT
<213> Artificial Sequence

<220>
<223> peptide synthesis product

<220>
<223> C terminus has linker-FITC attached

<400> 4

Arg Arg Arg Arg Arg Arg
1               5

<210> 5
<211> 10
<212> PRT
<213> Artificial Sequence

<220>
<223> peptide synthesis product

<400> 5
Arg Arg Arg Arg Arg Arg Glu Glu Glu Glu
1               5                   10

<210> 6
<211> 10
<212> PRT
<213> Artificial Sequence

<220>
<223> peptide synthesis product

<220>
<223> C terminus has linker-FITC attached

<400> 6
Arg Arg Arg Arg Arg Arg Glu Glu Glu Glu
1               5                   10

<210> 7
<211> 10
<212> PRT
<213> Artificial Sequence

<220>
<223> peptide synthesis product

<400> 7
Arg Arg Arg Arg Arg Arg Glu Glu Glu Glu
1               5                   10

## Description

The invention provides a transmucosal and transepithelial delivery system for transporting pharmaceutically active moieties across, for example, skin, the surface of the eye, or mucosal membranes. Methods of using the delivery systems are also provided.

There is a wide range of applications for delivering pharmaceutically active moieties across mucosal and epithelial surfaces. These include the treatment for acute conditions, such as bums, trauma and infection; and chronic treatments for conditions that include debilitating eye diseases such as glaucoma, age-related macular degeneration and retinal scarring after ocular surgery. To show proof of principle, we have focused on delivery of an anti-scarring drug into the eye to target one of the pathological processes occurring in Glaucoma.

Uemura et al (Circulation J. (2202), 66, 1155-1160) describe the translocation of short polymers of arginine into cultured vascular smooth muscle cells. Heptamers of arginine were especially rapidly transported into cells where they were used to study the effects of the polymers on nitric oxide synthesis. The polymers were observed to rapidly translo-cate through cytoplasmic and nuclear membranes efficiently.

Other so called cell penetrating peptides have also been used to transport, for example, DNA or siRNA into cultured cells, but not in a more complex system of several layers of cells as found in transmembrane systems.

The inventors have unexpectedly found that it is possible to transport molecules, such as the Decorin, across the dermis, cornea or skin of patients by using them in combination with a polypeptide having basic amino acids.

Glaucoma is a chronic neurodegenerative disease of the retina and optic nerve and is the second leading cause of blindness worldwide following cataracts (Resnikoff et al., 2004). However, unlike cataracts, left untreated glaucoma results in irreversible blindness (Weinreb and Khaw, 2004). Globally 66 million people have glaucoma and 6.8 million people are permanently blind in both eyes from this disease (Weinreb and Khaw, 2004).

Glaucoma's are a group of optic neuropathies which are characterised by progressive death of retinal ganglion cells (RGC) and their axons (which form the optic nerve) leading to visual defects and loss. The main, or most common, type of glaucoma is Primary Open Angle Glaucoma (POAG). Ocular hypertension, or increased intraocular pressure (IOP), is the main risk factor for the onset and exacerbation of POAG (Junglas et al., 2012) and, although the pathological process is not fully understood, it is thought that ocular hypertension leads to a continuous compression of axons at the optic nerve head leading to RGC death by apoptosis. Other risk factors for the development of POAG include increasing age, ethnicity and family history of glaucoma (Morrison & Pollack, 2002).

Pharmacological and surgical treatments for POAG focus solely on lowering IOP (to relieve compression on the RGC axons) as currently this is the only controllable risk factor, but does not address the underlying pathological process of glaucoma or provide any direct neuroprotection for RGC death (Marquis et al. 2005). Although some IOP lowering agents provide good symptomatic control, patient's vision deteriorates with time as RGC are lost, leading to the progressive blindness typical of the condition.

Although the aetiology of POAG is not understood, it is known that resistance to aqueous humour outflow through the TM increases IOP, which slowly leads to RGC death and degeneration of neurons in the lateral geniculate nucleus and the visual cortex. RGC degeneration occurs slowly from increased pressure on the lamina cribosa and RGC axons leading to altered cell morphology and death, by apoptosis, due to mechanical stresses blocking proteins and trophic factor transport along axons (Fraser, 2005; Weinreb and Khaw, 2004). The loss of RGC is correlated with loss of vision and it is surmised that prevention of RGC death by neuroprotection either directly using anti-apop-totic agents or indirectly by reducing outflow resistance will provide better treatments for patients.

The exact mechanisms which lead to resistance of outflow from the TM are not known. It is thought that structural changes within the TM leads to increased levels of extracellular matrix (ECM) deposition (Junglas et al., 2012). Scarring, or fibrosis, is a wound healing response following cellular or metabolic insults. Increased levels of TM fibrosis have been observed in patients and this is suggested to be responsible for the ocular hypertension seen in POAG (Borras, 2003; WuDunn, 2009). Increased ECM deposition within the eye occurs with age in the normal population but this process is thought to be more aggressive, and occur sooner, in patients with glaucoma (Tektas and Liitjen-Drecoll, 2009).

Aberrant growth factor signaling is thought to be responsible for excess ECM deposition and reduced ECM degradation within the TM and SC. Transforming growth factor (TGF)-β's are multifunctional cytokines that are up-regulated in response to inflammation and injury (Logan et al., 1992). TGF-β 1&2 are the main cytokines involved in scarring within the CNS (Logan et al., 1992) and are thought to play a role in glaucoma by increasing TM fibrosis (Shepard et al. 2010; Junglas et al, 2012). Higher levels of TGF-β were detected in aqueous humour samples of patients with POAG compared to non-glaucoma-tous controls (Gottanka, 2004). Other studies have shown that TGF-β antagonists (e.g. Decorin) prevent and reduce scar formation within the CNS (Logan et al., 1999). Hence antagonising the TGF-β sub 10 family of cytokines may be a promising avenue for a better treatment for glaucoma as the strategy targets the pathology rather than just the symptoms.

Decorin is a naturally occurring ECM dermatan sulphate glycoprotein of approx 70kD (Brandan et al., 1992). It interacts with ECM proteins, cytokines and cell surface receptors including TGF-β (Logan et al. 1999), epidermal growth factor receptor (Biglari et al., 2004) and vascular endothelial growth factor (Grant et al., 2002). It is found in tissue rich in fibrillar collagen where it plays an important role in its formation. Decorin sequesters TGF-βs and prevents them binding to their receptors and has been shown to reduce scar formation and inflammation following cerebral injury (Logan et al., 1999) and spinal cord damage (Davies et al, 2004).

The invention provides a transmembrane/transepithelial delivery system comprising a pharmaceutically effective moiety; and a polypeptide of up to 20 amino acids in length, polypeptide comprising a continuous region of at least 2, more Typically, at least 4 basic amino acids. Typically, the polypeptide comprises at least 4, at least 5, at least 6, at least 7, at least 8, at least 9, at least ten, or less than 19, less than 18, less than 17, less than 16, less than 15, less than 14, less than 13, less than 12, less than 11 or less than 10 amino acids in length.

Amino acids may be D or L amino acids, preferably L amino acids, and may be naturally or non-naturally occurring. That is, they may occur naturally within the cell or may be artificial or synthetic peptides.

Typically, the amino acids making the polypeptide are joined by means of peptidic bonds to each other.

The polypeptides may be covalently or non-covalently bound to a support, such as titanium or hydroxyapatite, or a polymer or hydrogel, for example by grafting onto the polymer or hydrogel or, for example, via a binding moiety comprising an amino acid sequence.

Typically, the polypeptide has formula:

(B)ₙ (A)m

where B is a basic amino acid
A may or may not be present and where present is an acidic amino acid, and m and n and integers and
n is at least 4.

Typically, m is less than n.

Typically, the total number of amino acids is 6, 7, 8, 9 or 10. n may be 6 and m may be 4, for example.

Typically, all of the amino acids are basic amino acids. 1, 2 or 3 non basic or non-acidic amino acids may also be present.

Basic amino acid may be selected from arginine, lysine and histidine, or mixtures thereof. Typically, the basic amino acid is arginine. Typically, the polypeptide is a polyarginine consisting of 6, 7 or 10 arginine residues.

The acidic amino acid may be selected from aspartate and glutamate. Typically, the acidic amino acid is glutamate. The acid amino acid residues may allow the polypeptide to bind to a support such as hydroxyapatite. The acidic amino acids may be replaced by an alternative amino acid sequence to allow it to bind to metals such as titanium or electrostatically to the pharmaceutically active moiety. The sequence may be, for example, RKLPDA to bind to titanium.

The polypeptide may be grafted or incorporated onto a polymer or hydrogel or similar material.

The pharmaceutically active moiety may be, for example but not exclusively, a polypeptide, proteins, a glycoprotein, or nucleic acids such as siRNA. It may, for example be an anti-inflammatory compound or an antimicrobial compound.

The polypeptide or glycoprotein is typically a negatively charged polypeptide or glycoprotein.

The pharmaceutically active moiety may have a molecular weight of up to, for example, 500 kDa, 30-400 kDa, 100-200 kDa or 150 kDa.

The polypeptide may form nanosomes of between 2 -5,000 nm, more typically 10 - 1,000 nm or 100 - 500 nm diameter. This is typically, when in combination with the pharmaceutically active moiety, so the total size of the polypeptide - pharmaceutically active complex is typically between 2-5,000nm, 10-1,000nm or 100-500nm diameter. Alternatively, the polypeptides themselves form nanosomes, which bind to larger pharmaceutically active moieties.

The pharmaceutically active moiety and polypeptide may, for example, be ionically bound together. Alternatively they may be covalently bound. They may be covalently bound, for example, through an attachment moiety, which covalently attaches the polypeptide and pharmaceutically active moiety. The attachment moiety may comprise: succinimidyl succinate, N-hydroxy succinimide, succinimidyl propionate, succinimidyl butanoate, propionaldehyde, acetaldehyde, tresylate, triazine, vinyl sulfone, benzotriazole carbonate, maleimide, pyridyl sulfide, iodoacetamide, succimidyl carbonate or avidin/biotin.

Such moieties are generally known in the art for the attachment of compounds to proteins or other pharmaceutically active moieties.

For example, the polypeptide may comprise an attachment moiety, optionally attached to the polypeptide via a linker, such as an alkyl moiety. The polypeptide may be then reacted with the pharmaceutically active moiety to covalently link the polypeptide to the pharmaceutically active moiety.

The system of the invention is suitable for transporting pharmaceutically active agents across single and multi-layered membranes, for example, the cornea of the surface of the eye, and indeed mucous membranes or indeed skin. Depending on the area to be treated, one or more additional compounds may be provided to assist with the penetration of the material or to make the material more suitable for the intended use.

For example, the system may be provided as in the form of eye drops, for example in a saline solution for applying to the surface of the eye to allow the pharmaceutically active moiety to be transported across the membrane of the eye into the eye itself.

Similarly, it may be provided in the form of drops, for example for applying to the mucous membrane of the nose, or indeed as an ointment for applying to the surface of the nose. The system may be provided in the form of a suppository to allow the pharmaceutically active agent to be absorbed through the mucous membranes of the rectum, vagina, or urethra. Typically, such suppositories comprise a greasy base, such as cocoa butter, in which the active ingredient and other excipients are dissolved. The grease will melt at body temperature to release the active ingredients. Other suppositories may be made from a water soluble base such as polyethylene glycol, glycerin or gelatin.

A system may also be provided in the form of a patch which is applied to the surface of the skin.

Such patches are generally known.

Transdermal patches are medicated adhesive patches that are placed on the skin to deliver a specific dose of medication through the skin and into the blood stream. The pharmaceutically active moiety and polypeptide may be provided within, for example, an adhesive layer. The adhesive layer not only serves to adhere various layers together, along with the entire system to the skin, but is also responsible for the releasing of the pharmaceutically active moiety and polypeptide onto the skin. Multi-layer drug systems are also known which use a separate layer for control of release of drug from a reservoir onto the skin.

The system of the invention may be used in combination with one or more additional penetration enhancers.

The penetration enhancer may be selected from but not exclusively, for example, polyethylene glycol, fatty acid esters, diacids and monomethyl esters.

The invention also provides the use of the systems of the invention in the treatment of diseases, such as eye diseases. The eye diseases include, for example, the treatment of glaucoma, proliferative vitreoretinopathy and age related macular degeneration scarring. It may also be used to treat bums and other wounds on both the eye and skin.

Methods of treating disease comprising applying a pharmaceutically effective amount of a system of the invention is also provided.

The invention also provides a method of transporting a pharmaceutically effective moiety across skin, the surface of an eye or a mucous membrane, comprising applying to the skin or membrane a system according to the invention.

In one embodiment, the present invention relates to a transmembrane delivery system comprising:
a pharmaceutically active moiety; and
a polypeptide of up to 20 amino acids in length comprising a continuous region of at least 2, more typically at least 4 basic amino acids.

Preferably, the polypeptide forms nanosomes of typically 2-5,000 nm.

In a further preferred embodiment or more preferably, the basic amino acids are linked to an amino acid sequence defining a binding moiety, most preferably a metal binding moeity.

In a further preferred embodiment or more preferably, the polypeptide has the formula:

(B)n (A)m

where B is a basic amino acid
A is an acidic amino acid, and
m and n and integers and
n is at least 4
m is less than n.

In a further preferred embodiment or more preferably, the polypeptide consists of basic amino acids.

In a further preferred embodiment or more preferably, the basic amino acid is selected from arginine (R), lysine (K) and histidine (H).

The acidic amino acid is preferably selected from aspartate (D) and glutamate (E).

In a further preferred embodiment or more preferably, the pharmaceutically active moiety is a polypeptide, a glycoprotein or nucleic acids such as siRNA.

In a further preferred embodiment or more preferably, the pharmaceutically active moiety has a molecular weight of up to 500 kDa, preferably the pharmaceutically active moiety is Decorin.

In a further preferred embodiment or more preferably, the pharmaceutically active moiety and polypeptide are covalently bound or non-covalently bound, preferably the polypeptide is attached to the pharmaceutically active moiety via an attachment moiety which covalently attaches the polypeptide and pharmaceutically active moiety, the attachment moiety comprising: succinimidyl succinate, N-hydroxy succinimide, succinimidyl propionate, succinimidyl butanoate, propionaldehyde, acetaldehyde, tresylate, triazine, vinyl sulfone, benzotriazole carbonate, maleimide, pyridyl sulfide, iodoacetamide, succimidyl carbonate, maleimidyl or avidin/biotin.

In a further preferred embodiment or more preferably, the system additionally comprises one or more penetration enhancers selected from polyethylene glycol, fatty acid esters, diacids or monomethyl esters.

In a further preferred embodiment or more preferably, the system is adapted to be applied to eyes or mucous membranes.

In a further preferred embodiment or more preferably, the system is in the form of a skin patch, eye drops, nose drops or a suppository.

In a further preferred embodiment or more preferably, the system is for use in the treatment of disease, wherein the disease is preferably eye disease.

In a further embodiment, the invention relates to a method of treating disease comprising applying a pharmaceutically effective amount of the transmembrane delivery system according to the present invention.

In a further embodiment, the invention relates to a method of transporting a pharmaceutically active moiety across skin, the surface of an eye or a mucose membrane comprising applying to the skin, surface of the eye or membrane the transmembrane delivery system according to the present invention.

The invention will now be described by way of example only with reference to the following figure:
**Figure 1** shows an initial ELISA data for the passage of Decorin across the cornea when aided by the nanosome.
**Figure 2** shows OCT images of nanosome/Decorin crossing the eye.
**Figure 3** shows TEM image of nanosome drops.
**Figure 4** shows Decorin concentration after treatment of nanosome drops to the cornea of rats.

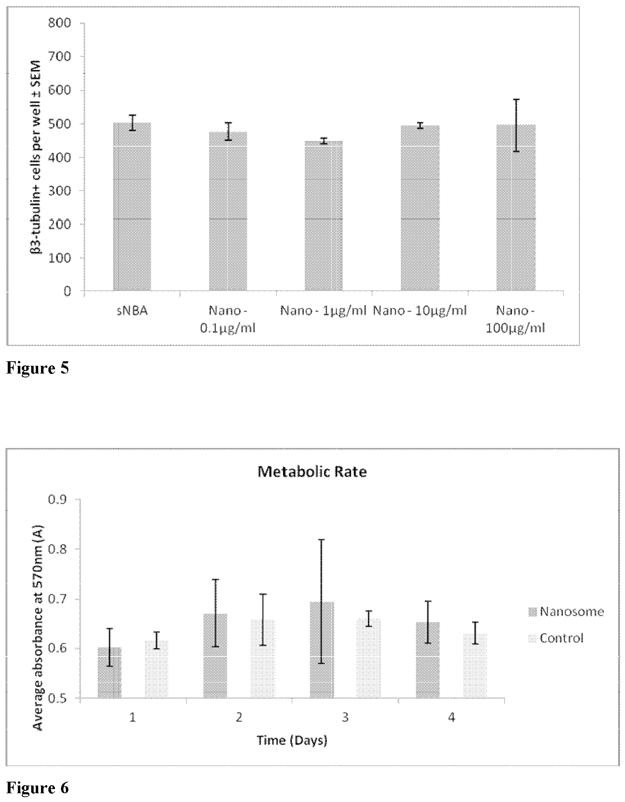

**Figure 5** shows cytotoxic effects of nanosomes against Retinal Ganglion Cells.
**Figure 6** shows metabolic activity of MF cells when treated with nanosome.
**Figure 7** shows DNA assay on cell samples to give viability.
**Figure 8** shows CD data for nanosome peptide.
**Figure 9** shows the concentration of avastin in eye after treatment with saline (PBS), nanosomes, avastin and nanosomes combined with avastin on application to enucleated pig eyes.

### Methods

Peptides were synthesised using the solid phase peptide synthesis method (SPPS). This used resin beads as a solid phase support and Fmoc (Fluorenylmethyloxycarbonyl) protection chemistry, which ensured that the correct reactions occurred. Wang beads were preattched with an initial amino acid on the bead. All the amino acids were protected on the amine group with an Fmoc protection group. The Fmoc group was cleaved using piperidine which gave the free amine group on the amino acid. Once the Fmoc group was removed a second Fmoc protected amino acid was added to the resin. This coupling reaction uses HBTU (*O*- (Benzotriazol-1-yl)-*N*,*N*,*N'*,*N'*-tetramethyluronium hexafluorophosphate) as a coupling reagent and was facilitated by DIPEA (diisopropylethylamine) which activated the carboxyl group and increased the reaction speed. This reaction gave rise to amide bond formation between the two amino acids and a dipeptide with a protected amine terminus was formed. This was then deprotected and reacted further until the full amino acid sequence was achieved. This peptide synthesis method has been utilised to create several different peptides, 1) RRRRRRRRRR, 2) RRRRRR, 3) RRRRRR-Linker-FITC, 4) RRRRRR-EEEE, 5) RRRRRR-EEEE-Linker-FITC. The peptides were analysed using mass spectrometry, which demonstrated the presence of the peptides in each group and by HPLC to ascertain the purity of the peptides. Covalent Decorin/nanosome drops were synthesised using a maleimide linker to crosslink the amine group of the nanosome with a thiol group on the Decorin protein sequence.

Spectroscopy was used to try and determine the binding interactions between Decorin and the RRRRRR-Linker-FITC. The spectra of Decorin was monitored using UV-Vis spectroscopy. The nanosomes were titrated into the Decorin solution with continuous UV monitoring. Peak shifts were observed on the addition of 2×10⁻⁷ M.

**Table 1 UV shift of nanosome-Decorin binding**

| Decorin | Decorin + nanosome |
|---|---|
| | |
| 208.5 | 203.5 |
| 205.5 | 200.5 |

This shift demonstrated that the nanosomes were binding with the Decorin. The shift pattern suggested that the peptide: Decorin was binding in a 2:1 ratio.

The RRRRRR-Linker FITC peptide was then taken forward for *in vitro* testing.

### Initial in-vivo testing

5mg of nanosome (NS) was diluted in 500ul PBS and 5mg of NS diluted in 500ul 5mg/ml Decorin.

Four male Sprague Dawley rats were given 3 drops to each eye. All left eyes were given PBS drops and all right eyes given Decorin drops. Two minutes after rats were killed using increasing concentrations of C02 over 8 minutes. 10ul of Aqueous humour (AqH) was removed from the anterior segment of each eye using glass micropipettes and placed into Eppendorf tubes ready for analysis. All AqH from eyes treated with PBS were pooled together to a final volume of 40ul. All AqH from eyes treated with Decorin were pooled together to a final volume of 40ul.

The AqH samples were analysed to ascertain Decorin concentrations using the commercially available human Decorin ELISA kit (R&D Systems, Cat: DY143). The ELISA protocol was as per kit instructions. No Decorin was detected in the AqH sample following external NS+PBS drops. Following the external NS+Decorin drop, 6.47ng/40ul of Decorin was detected in the AqH.

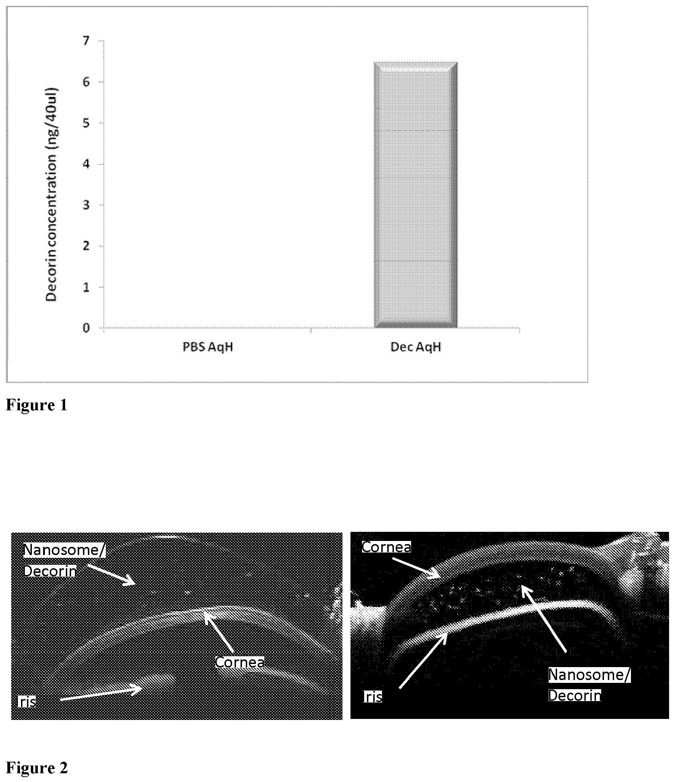

As Figure 1 shows, when nanosome + Decorin drops are applied to the front of the cornea. Decorin is rapidly found in the aqueous humour. The level of Decorin found in the aqueous humour is significantly higher than the control. This demonstrates that the Decorin is being carried across the cornea and into the eye. This experiment utilised the electrostatic drop with no covalent linkage between the nanosome and Decorin.

As Figure 2 demonstrates when the nanosome /Decorin mixture is applied to the eye, it diffuses across the cornea and into the aqueous humour. In the first image, the nanosome/Decorin drop can be seen as a liquid droplet on the front of the cornea at time 0. After 6 minutes the fluorescently tagged nanosome/Decorin can be seen in the anterior chamber. As the nanosome/Decorin can be seen as small dots visible to the eye it was hypothesised that the nanosome/Decorin was aggregating together in structures big enough to be visible on the OCT. Dynamic light scattering (DLS) was carried out on the solution to find out if structures were forming. The DLS demonstrated a range of sizes of particles from 2nm to 5000nm. This showed that the nanosome + Decorin were forming aggregates with each other but there did not appear to be any specific size to the aggregates and this did not affect the bioactivity or clearance.

The aggregates were imaged using Transmission Electron Microscopy (TEM). This showed a number of aggregates of different sizes on the micro and the nanoscale. There was no specific order to the structures and a broad range of sizes were seen. These structures probably occur as the nanosome peptides bind more than one molecule of Decorin at a time bringing them into close proximity and causing aggregation.

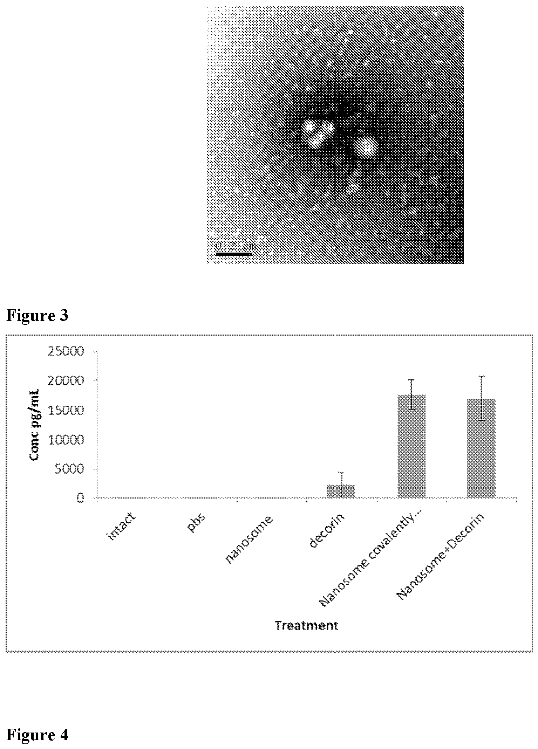

A second ELISA test was then carried out to determine levels of Decorin carried across the cornea. This was to compare the electrostatic and the covalent drop and to cover all controls (Figure 4).

Figure 4 shows that using the electrostatic drop where the nanosome and the Decorin are not covalently cross linked does significantly increase the level of Decorin which passes across the cornea from the topical application.

The cells were studied against several cell lines for any cytotoxic effects they might induce. Dissociated retinal cultures were prepared from rats and plated onto culture plates. The cells were treated with nanosome peptide and then incubated for 72 hours and fixed with formaldehyde. The cells were stained with β3 tubulin and imaged on the fluorescence microscope. The positive cells were counted and comparisons were taken. The data shows that there is no significant difference between wells treated with the nanosome peptide and the control and that increasing the nanosome peptide concentration upto 100 µg/mL did not have any effect on cell viability.

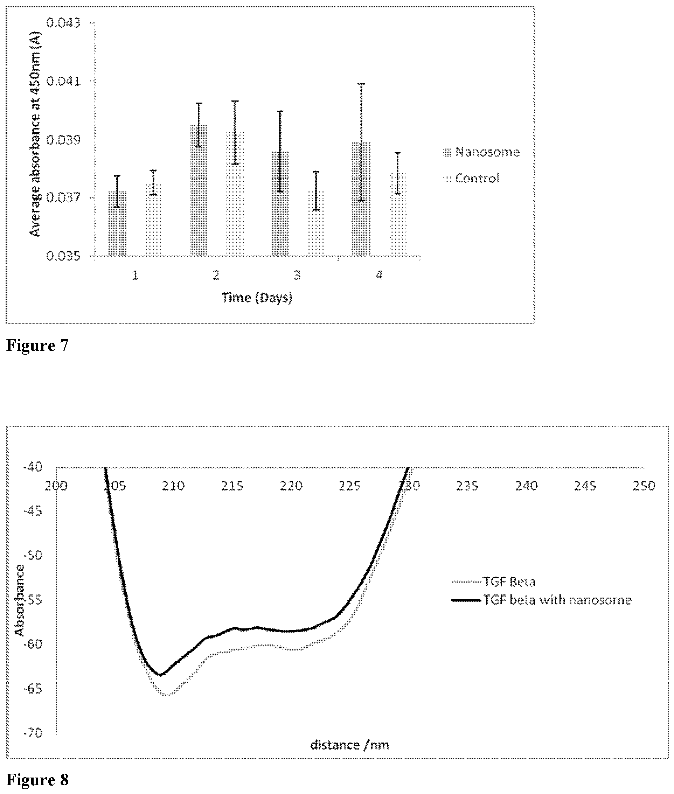

The nanosome toxicity was also tested against primary rat meningeal fibroblast cells. The cells were isolated from Sprague-Dawley Rats. The leptomeninges was removed from the rat and the meningeal fibroblast cells were isolated from the tissue. The cells were then plated out and treated with nanosome. The cell media was supplemented with resazurin for 2 hours and then harvested and the media fluorescence read at 570 nm. The cell samples were then frozen in deionised water for DNA analysis (Figure 7).

Figure 7 shows that the nanosome peptide did not have a significant effect on the number of cells in the sample, with no significant differences observed between the peptide and the control. This supports the work carried out in the retinal cultures that the nanosomes are not toxic.

Decorin interacts with TGF β and other bioactive molecules in tissues to modulate their activity and to provide a clinical result. In order to understand if there are any interactions between nanosomes and TGF beta, circular dichroism was carried out to monitor structural shifts in TGF beta in the presence of the peptide. A slight shift is seen when the peptide is titrated into the TGF beta solution. This indicates that the nanosome could induce a structural change in the TGF beta. As TGF beta and nanosomes are both positively charged this cannot be due to electrostatics and suggests that the nanosomes are targeting a specific site on the TGF beta molecule.

Further studies utilising the nanosome also show that it is a highly effective broad spectrum antibacterial agent. It is effective at killing both gram positive and gram negative bacteria and was particularly selective against pseudomonas aeruginosa with a MIC of 0.03 mg/mL.

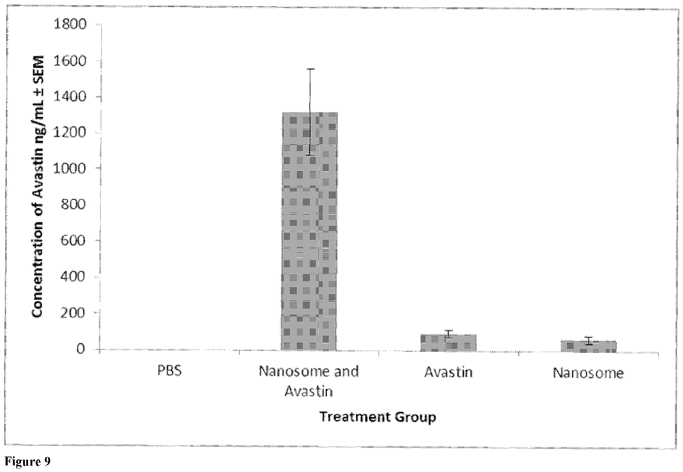

Further studies looked at the transport of avastin across pig eyes. Freshly enucleated pig eyes and had applied nanosomes, avastin, nanosomes and avastin or saline to the ocular surface. These were left for two hours and the inventors then removed the vitreous and retina. They then used an ELISA to measure the concentration of the avastin in the vitreous. Concentrations are shown in Figure 9.
Resnikoff S, Pascolini D, Etya'ale D, Kocur I, Pararajasegaram R, Pokharel GP, Mariotti SP. Global data on visual impairment in the year 2002. B World Health Organ. 2004;82:844-851
Weinreb RN, T KP. Primary open-angle glaucoma. Lancet. 2004;363:1711-1720
Junglas B, Kuespert S, Seleem AA, Struller T, Ullmann S, Bosl M, Bosserhoff A, Kostler J, Wagner R, Tamm ER, Fuchshofer R. Connective tissue growth factor causes glaucoma by modifying the actin cytoskeleton of the trabecular meshwork. The American Journal of Pathology. 2012;180:2386-2403
Morrison JC, Pollack IP. Glaucoma: Science and practice. New York: Thieme; 2003.
Marquis RE, Whitson JT. Management of glaucoma: Focus on pharmacological therapy. Drugs & Aging. 2005;22:1-21 Fraser 2005
Borras T. Gene expression in the trabecular meshwork and the influence of intraocular pressure. Progress in Retinal and Eye Research. 2003;22:435-463
WuDunn D. Mechanobiology of trabecular meshwork cells. Experimental Eye Research. 2009;88:718-723
Tektas OY and Lutjen-Drecoll E. Structural changes of the Trabecular Meshwork in different kinds of glaucoma. Experimental Eye Research. 2009; 88:769-775
Logan A, Frautschy SA, Enhanced expression of transforming growth factor [beta] 1 in the rat brain after a localized cerebral injury. Brain Research. 1992; 587:216-225
Gottanka J, Chan D, Eichhorn M, Lutjen-Drecoll E, Ethier CR. Effects of tgf-(32 in perfused human eyes. Invest Ophth Vis Sci. 2004;45:153-158
Logan A, Baird A, Berry M. Decorin attenuates gliotic scar formation in the rat cerebral hemisphere. Experimental Neurology. 1999;159:504-510
Brandan E, Fuentes ME, Andrade W. Decorin, a chondroitin/dermatan sulfate proteogly-can is under neural control in rat skeletal muscle. Journal of Neuroscience Research. 1992;32:51-59
Biglari A, Bataille D, Naumann U, Weller M, Zirger J, Castro MG, Lowenstein PR. Effects of ectopic decorin in modulating intracranial glioma progression in vivo, in a rat syngeneic model. Cancer Gene Ther. 2004;11:721-732
Grant DS, Yenisey C, Rose RW, Tootell M, Santra M, lozzo RV. Decorin suppresses tumor cell-mediated angiogenesis. Oncogene. 2002;21:4765-4777Davies et al 2004

## Claims

1. A transmembrane, transmucosal or transepithelial delivery system for use in a method of treating an eye disease, wherein the system comprises:
a pharmaceutically active agent which is a polypeptide, a protein or a glycoprotein;
and
a polyarginine of 4 to 20 amino acids in length;
wherein the pharmaceutically active agent is mixed with or non-covalently bound to the polyarginine.

2. The system for use according to claim 1, wherein the polyarginine consists of 6, 7 or 10 arginine residues.

3. The system for use according to claim 1 or 2, wherein the pharmaceutically active agent has a molecular weight of up to 500 kDa.

4. The system for use according to any one of claims 1 to 3, wherein the pharmaceutically active agent is an anti-inflammatory compound, an anti-scarring drug or an antimicrobial compound.

5. The system for use according to any one of claims 1 to 4, which further comprises or is used in combination with one or more penetration enhancers, such as polyethylene glycol, fatty acid esters, diacids and/or monomethyl esters.

6. The system for use according to any one of claims 1 to 5, which is applied to the eye via an eye drop.

7. The system for use according to any one of claims 1 to 6, which transports the pharmaceutically active agent across a surface of the eye.

8. The system for use according to any one of claims 1 to 7, wherein the eye disease is age-related macular degeneration, proliferative vitreoretinopathy, glaucoma or retinal scarring after ocular surgery.

9. A transmembrane, transmucosal or transepithelial delivery system for application to the eye, wherein the system comprises:
a pharmaceutically active agent which is a polypeptide, a protein or a glycoprotein;
and
a polyarginine of 4 to 20 amino acids in length;
wherein the pharmaceutically active agent is mixed with or non-covalently bound to the polyarginine.

10. The system for application according to claim 9, wherein the polyarginine consists of 6, 7 or 10 arginine residues.

11. The system for application according to claim 9 or 10, wherein the pharmaceutically active agent has a molecular weight of up to 500 kDa.

12. The system for application according to any one of claims 9 to 11, wherein the pharmaceutically active agent is an anti-inflammatory compound, an anti-scarring drug or an antimicrobial compound.

13. The system for application according to any one of claims 9 to 12, which further comprises or is used in combination with one or more penetration enhancers, such as polyethylene glycol, fatty acid esters, diacids and/or monomethyl esters.

14. The system for application according to any one of claims 9 to 13, which is applied to the eye via an eye drop.

15. The system for application according to any one of claims 9 to 14, which transports the pharmaceutically active agent across a surface of the eye.
